(19) Europäisches Patentamt
European Patent Office
Office européen des brevets

(11) Veröffentlichungsnummer: **0 578 665 B1**

(12) **EUROPÄISCHE PATENTSCHRIFT**

(45) Veröffentlichungstag der Patentschrift: **21.12.94**

(51) Int. Cl.$^5$: **A61K 7/13**

(21) Anmeldenummer: **92907054.8**

(22) Anmeldetag: **27.03.92**

(86) Internationale Anmeldenummer:
**PCT/EP92/00681**

(87) Internationale Veröffentlichungsnummer:
**WO 92/17156 (15.10.92 92/26)**

# (54) HAARFÄRBEMITTEL MIT DIREKTFARBSTOFFEN.

(30) Priorität: **05.04.91 DE 4110995**

(43) Veröffentlichungstag der Anmeldung:
**19.01.94 Patentblatt 94/03**

(45) Bekanntmachung des Hinweises auf die Patenterteilung:
**21.12.94 Patentblatt 94/51**

(84) Benannte Vertragsstaaten:
**AT BE CH DE DK ES FR GB GR IT LI NL SE**

(56) Entgegenhaltungen:
**EP-A- 0 220 613**
**EP-A- 0 280 187**

(73) Patentinhaber: **Henkel Kommanditgesellschaft auf Aktien**

**D-40191 Düsseldorf (DE)**

(72) Erfinder: **LIESKE, Edgar**
**Hunsrückenstr. 40**
**D-4000 Düsseldorf (DE)**
Erfinder: **ROSE, David**
**Am Eichelkamp 223**
**D-4010 Hilden (DE)**
Erfinder: **GIEDE, Karl**
**Schlehenweg 12**
**D-4010 Hilden (DE)**
Erfinder: **HÖFFKES, Horst**
**Carlo-Schmid-Str. 113**
**D-4000 Düsseldorf 13 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Gegenstand der Erfindung sind Haarfärbemittel mit einem Gehalt an wenigstens zwei verschiedenen direktziehenden Haarfarbstoffen, die sich durch einen besonders gleichmäßigen Farbaufzug, auch auf geschädigtes Haar auszeichnen.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein. Leider haben viele direktziehende Haarfarbstoffe den Nachteil, daß sie nur ungleichmäßig auf das Haar aufziehen, und zwar wird der stärker geschädigte Bereich der Haarspitze meist intensiver angefärbt als der jüngere und weniger geschädigte Bereich des Haaransatzes. Aus diesem Grunde sind viele an sich gute Farbstoffe für den praktischen Einsatz ungeeignet.

Es wurde nun gefunden, daß Kombinationen von zwei oder mehreren direktziehenden Farbstoffen eine wesentlich gleichmäßigere Anfärbung auch von Haaren ermöglichen, die durch reduktive und oxidative Haarbehandlung, z. B. durch Dauerwellen und Bleichen, geschädigt sind, wenn in dieser Kombination wenigstens ein Nitrodiphenylamin-Farbstoff der Formel I enthalten ist.

Gegenstand der Erfindung sind demgemäß Haarfärbemittel mit einem Gehalt an wenigstens zwei direktziehenden Farbstoffen in einem kosmetischen Träger, die einen ersten direktziehenden Farbstoff (A) der Formel I

(I) 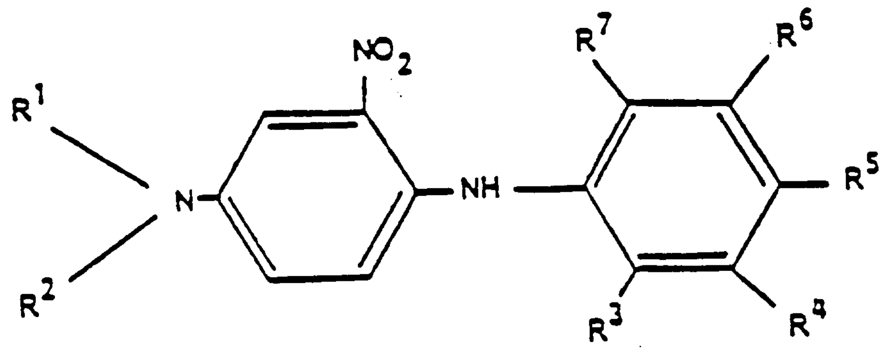

in der $R^1$ und $R^2$ Wasserstoff, Alkylgruppen mit 1 bis 4 C-Atomen oder Hydroxyalkylgruppen mit 2 bis 4 C-Atomen und eine der Gruppen $R^3$ bis $R^7$ eine $-SO_3H$ oder eine -COOH-Gruppe ist und die anderen Wasserstoff, Chlor, Alkylgruppen mit 1 bis 4 C-Atomen, Alkoxygruppen mit 1 bis 4 C-Atomen oder Gruppen der Formel $-NR^8R^9$ sind, worin $R^8$ und $R^9$ Wasserstoff, Alkylgruppen mit 1 bis 4 C-Atomen, Hydroxyalkylgruppen mit 2 bis 4 C-Atomen sind oder gemeinsam mit dem Stickstoffatom einen Piperidin-, Morpholin-, Piperazin- oder Pyrrolidinring bilden, oder eines seiner wasserlöslichen Salze und wenigstens einen zweiten direktziehenden Farbstoff (B) einer anderen Struktur enthalten.

Die direktziehenden Farbstoff (A) der Formel I sind aus der Europäischen Patentanmeldung EP 0 280 187 A1 bekannt. Durch die Anwesenheit dieser Farbstoffe wird die Gleichmäßigkeit des Farbaufzugs zahlreicher anderer direktziehender Farbstoffe deutlich besser als es aus dem Farbaufzug der einzelnen Farbstoffe für die Kombination erwartet werden kann. In den meisten Fällen zieht die Kombination aus dem Farbstoff (A) der Formel I und einen zweiten Farbstoff (B) gleichmäßiger auf als jede der Komponenten (A) und (B) für sich. Zumindest aber liegt die Gleichmäßigkeit des Farbaufzuges höher als es der Mischungsregel entspricht.

Als zweiter Farbstoff (B) können direktziehende Farbstoffe verschiedener Struktur eingesetzt werden. Besonders bevorzugt sind direktziehende Farbstoffe aus der Gruppe der aromatischen Nitroverbindungen oder der Anthrachinonverbindungen. Die erfindungsgemäße Wirkung einer verbesserten Gleichmäßigkeit des Farbaufzugs durch Kombination mit Farbstoffen der Formel I wird jedoch auch bei anderen direktziehenden Farbstoffen, z. B. bei Azofarbstoffen, Triphenylmethanfarbstoffen oder Indophenolen beobachtet.

Besonders geeignete direktziehende Farbstoffe (B) sind im Beispielteil, insbesondere gemäß B1 bis B9, aufgeführt.

Besonders geeignete direktziehende Nitrodiphenylamin-Farbstoffe (A) der Formel I sind

A1: 2-Nitro-4-aminodiphenylamin-2'-carbonsäure und

A2: 2-Nitro-4-amino-4'-dimethylaminodiphenylamin-2'-carbonsäure

Weitere Beispiele sind aus der Europäischen Patentanmeldung EP 0 280 187 A1 zu entnehmen.

Die erfindungsgemäßen Haarfärbemittel zeichnen sich auch durch eine verbesserte Waschechtheit aus. Färbungen, die mit einer Kombination aus einem Nitrodiphenylamin-Farbstoff (A) der Formel I und einem zweiten direktziehenden Farbstoff (B) einer anderen Struktur erzeugt wurden, werden beim Haarewaschen weniger stark aufgehellt, als solche, die mit dem Farbstoff (B) in Abwesenheit eines Farbstoffs der Formel I erhalten werden.

Die erfindungsgemäßen Haarfärbemittel können auch zwei verschiedene Farbstoffe (A) der Formel I und einen oder mehrere weitere Farbstoffe (B) anderer Struktur enthalten.

Schließlich können die erfindungsgemäßen Haarfärbemittel auch Oxidationsfarbstoffvorprodukte enthalten.

Zur Herstellung der erfindungsgemäßen Haarfärbemittel werden die direktziehenden Haarfarbstoffe (A) der Formel I und die weiteren Farbstoffe (B) in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische, kationische, zwitterionische und ampholytische Tenside. Bevorzugte Tenside sind z. B. Fettalkoholsulfate, Fettalkoholpolyglycolethersulfate, n-Alkansulfonate, $\alpha$-Olefinsulfonate, Ethylenoxidanlagerungsprodukte an Fettamine, an Fettsäuren, an Fettalkohole, an Alkylphenole, an Fettsäurepartialglyceride, an Sorbitanfettsäureester und an Fettsäurealkanolamide.

Weitere übliche Bestandteile sind wasserlösliche Verdickungsmittel, wie z. B. Methyl- oder Hydroxyethylcellulose, Stärke und wasserlösliche Stärkederivate, Biopolymere, wasserlösliche synthetische Polymere und Copolymere, z. B. solche der Acryl- und Methacrylsäure. Zur Formulierung als Färbecreme werden Fettkomponenten wie z. B. Fettalkohole, Fettsäuren, Fettsäurepartialglyceride, Ester, Paraffine und Wachse in emulgierter Form als Träger verwendet. Die Haarfärbemittel können darüber hinaus bekannte haarpflegende Zusätze enthalten wie z. B. wasserlösliche kationische Polymere, wasselösliche Proteine und Proteinderivate, Pantothensäure, Vitamine, Pflanzenextrakte sowie Duftstoffe, pH-Regulatoren (Puffersubstanzen), Elektrolyte und Wasser.

Die Bestandteile des Trägers werden zur Herstellung der erfindungsgemäßen Haarfärbemittel in für diese Zwecke üblichen Mengen verwendet, z. B. werden Netz- und Emulgiermittel in Mengen von 0,5 bis 30 Gew.-% und Verdickungsmittel in Konzentrationen von 0,1 bis 10 Gew.-% des gesamten Färbemittels eingesetzt.

In einer bevorzugten Ausführung ist der Träger eine Emulsion mit einem Gehalt von 1 - 5 Gew.-% eines Fettalkohols mit 12 - 22 C-Atomen, 5 - 20 Gew.-% eines Anlagerungsproduktes von 5 - 20 Mol Ethylenoxid an ein Mol eines linearen, endständigen Alkylamins mit 12 - 22 C-Atomen, jeweils bezogen auf das Gewicht des gesamten Haarfärbemittels.

In den erfindungsgemäßen Haarfärbemitteln werden die direktziehenden Haarfarbstoffe insgesamt (A + B) in einer Menge von 0,05 - 5 Gew.-%, bevorzugt von 0,1 - 2 Gew.-%, bezogen auf das gesamte Haarfärbemittel eingesetzt. Dabei kann das Mengenverhältnis der Farbstoffe (A) der Formel I zu den Farbstoffen (B) anderer Struktur im Bereich von 1 : 9 bis 9 : 1, bevorzugt im Bereich von 3 : 7 bis 7 : 3, liegen.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 6 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar

entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern, ohne ihn jedoch hierauf zu beschränken.

## Beispiele

1. Verbesserung des Egalisiervermögens

1.1 Herstellung der Färbezubereitungen

Es wurden Haarfärbemittel der folgenden Zusammensetzung hergestellt:

| | |
|---|---|
| Talgalkylamin ($C_8$-$C_{18}$)-polyglycol(8 EO)-ether | 9,0 g |
| Ölsäurepolyglycerinester | 2,5 g |
| Cetyl-/Oleylalkohol-polyglycolether (5 EO) | 3,0 g |
| Propylenglycol-1,3 | 5,0 g |
| Direktzieher (oder Direktzieherkombination) | 1,0 g |
| Duftstoff | 0,3 g |
| $(NH_4)_2SO_4$ | 1,0 g |
| Ammoniak (konz. in $H_2O$) bis pH = 9 | |
| Wasser | ad 100 g |

Als Direktzieher der Formel I wurden eingesetzt:

A1 : 2-Nitro-4-amino-2'-carboxydiphenylamin
A2 : 2-Nitro-4-amino-2'-carboxy-4'-dimethyl-amino-diphenylamin

Als weitere Direktzieher wurden eingesetzt:

B 1 : 1,2,3,4-Tetrahydro-6-nitrochinoxalin
B 2 : Pikraminsäure (2-Amino-4,6-dinitrophenol)
B 3 : 1-Amino-2-nitro-4-(2,3-dihydroxypropylamino)-5-chlorbenzol
B 4 : 1-Amino-2-nitro-4(2-hydroxyethylamino)-5-chlorbenzol
B 5 : 1,4-Bis-($\beta$-hydroxyethylamino)-2-nitrobenzol
B 6 : 4-(3-Hydroxypropyl)amino-3-nitrophenol
B 7 : 1-(2-Hydroxyethyl)amino-4-methyl-2-nitrobenzol
B 8 : 6-Chlor-4-nitro-2-aminophenol
B 9 : 1-(2-Hydroxyethyl)-amino-2-nitro-4-bis-(2-hydroxy-ethyl)-aminobenzol
B10 : 1-(2-Hydroxyethyl-)amino-2-nitro-4-aminobenzol
B11 : 2-Nitro-p-phenylendiamin
B12 : 1-Amino-2-nitro-4-bis-(2-hydroxyethyl)-aminobenzol
B13 : 2-Amino-4-nitrophenol
B14 : 1-Amino-2-(2-hydroxyethyl)-amino-5-nitrobenzol
B15 : 1-Amino-2-nitro-4-(2-hydroxyethyl)-aminobenzol
B16 : 1,4,5,8-Tetraamino-anthrachinon
B17 : 1-Amino-4-methylamino-anthrachinon
B18 : 1,4-Diamino-5-nitro-anthrachinon
B19 : 1-Methylamino-4-(2-Hydroxylethyl)-amino-anthrachinon
B20 : 1,4-Diaminoanthrachinon
B21 : 1-(4'-Bis-(2-hydroxyethyl)-amino)-phenylazo-4'-aminobenzol

1.2 Haaranfärbung

Graue Haarsträhnen von 2 g Gewicht und einer Länge von 16 - 18 cm wurden im Bereich der Haarspitze (obere Hälfte) mit eines Kaltwellmittel (wäßrige Lösung von Ammoniumthioglycolat) 30 Minuten bei 27 °C behandelt, mit warmen Wasser gespült und danach mit 10 ml einer Fixierlösung (Kaliumbromat-Lösung) behandelt und wieder gespült. Dann wurde die gleiche Hälfte mit einer wäßrigen Zubereitung von Wasserstoffperoxid und Ammoniumperoxiddisulfat bei 27 °C 30 Minuten lang blondiert. Anschließend erfolgte noch einmal eine Behandlung mit dem Kaltwellmittel und der Fixierlösung. Schließlich wurde die ganze Haarsträhne einmal blondiert. Auf diese Weise wurden zwei

unterschiedlich strapazierte Bereiche, ein stark strapazierter Haarspitzen-Bereich und ein weniger strapazierter Haaransatz-Bereich, erhalten.

Die so vorbehandelten Strähnen wurden mit den Haarfärbemitteln gemäß 1.1 angefärbt, wobei als Direktzieher jeweils die in Tabelle I angegebenen Farbstoffe oder Farbstoffkombinationen eingesetzt wurden. Die Färbelösungen wurden ca. 20 Minuten bei 27 °C auf den Haarsträhnen belassen, dann mit einem üblichen Haarwaschmittel ausgewaschen, mit Wasser gespült und getrocknet.

1.3 Ermittlung der Gleichmäßigkeit der Haaranfärbung (Farbabstandswerte (DE) zwischen Haarspitze und Haaransatz)

Jede Haarsträhne wurde an acht Stellen (4 im Bereich des Haaransatzes und 4 im Bereich der Haarspitze mit Hilfe eines Farbmeßsystems der Firma Datacolor vermessen. Dabei wurde die zu vermessende Probe in einer Einspannvorrichtung am Spektralphotometer fixiert und die Remissionswerte über den Bereich des sichtbaren Lichtes von 390 - 700 nm im Abstand von 10 nm gemessen und über einen Rechner (Minicomputer HP 2113 E) verarbeitet. Das Rechnerprogramm ermittelte die Normfarbwerte nach dem CIE-System (Comission Internationale de l'Eclairage) entsprechende DIN 5033 und rechnete sie in Farbabstandszahlen nach DIN 6174 um.

In der Tabelle I sind die Farbabstands-Werte (DE) für die Ausfärbungen mit einzelnen Farbstoffen und mit erfindungsgemäßen Farbstoff-Kombinationen aufgeführt. Die Farbabstände der Ausfärbungen mit den erfindungsgemäßen Farbstoffkombinationen (Direktzieher Typ A + Typ B) sind ebenfalls der Tabelle zu entnehmen.

Tabelle I

| Direktzieher, Menge | Farbnuance Haarspitze | Farbnuance Haaransatz | Farb-Abstand DE |
|---|---|---|---|
| A1 : 1 g | graurot | violettbraun | 12,57 |
| A2 : 1 g | grauindigo | indigo | 21,1 |
| B1 : 1 g | braunrot | orange | 18,35 |
| B1 (0,5g) + A1 (0,5g) | rotbraun | rotbraun | 6,25 |
| B2 : 1 g | hellorangebraun | braunorange | 10,2 |
| B2 (0,5g) + A1 (0,5g) | rotbraun | rotbraun | 6,14 |
| B3 : 1 g | rotbraun | graurot | 9,4 |
| B3 (0,5g) + A1 (0,5g) | granatbraun | graurot | 2,8 |
| B4 : 1 g | himbeerrot | braunrot | 5,8 |
| B4 (0,5g) + A1 (0,5g) | rotbraun | graurot | 5,0 |
| B5 : 1 g | dunkelpurpur | graumagenta | 16,7 |
| B5 (0,5g) + A1 (0,5g) | rubin | graurot | 10,5 |
| B5 : 1 g | dunkelpurpur | graumagenta | 16,7 |
| B5 (0,5g) + A2 (0,5g) | graumagenta | magenta | 4,8 |
| B6 : 1 g | korallenrot | hummerrot | 4,3 |
| B6 (0,5g) + A1 (0,5g) | pompejenischrot | graurot | 3,5 |
| B7 : 1 g | gelborange | butterblumen-gelb | 11,8 |
| B7 (0,5g) + A1 (0,5g) | kaneelbraun | fuchsrot | 12,7 |
| B8 : 1 g | rotorange | melonenorange | 18,1 |
| B8 (0,5g) + A1 (0,5g) | terra di Siena | kupferrot | 8,4 |

Fortsetzung Tabelle I

| Direktzieher, Menge | Farbnuance Haarspitze | Farbnuance Haaransatz | Farb-Abstand DE |
|---|---|---|---|
| B9 : 1 g | graumagenta | graurubin | 5,75 |
| B9 (0,5g) + A1 (0,5g) | graurubin | mattviolett | 5,80 |
| B10 : 1 g | mattrot | graurot | 12,56 |
| B10 (0,5g) + A1 (0,5g) | violettbraun | violettbraun | 3,5 |
| B11 : 1 g | rotbraun | orangebraun | 19,9 |
| B11 (0,5g) + A1 (0,5g) | braunrot | braunrot | 3,88 |
| B12 : 1 g | graumagenta | hellbraun | 11,75 |
| B12 (0,5g) + A1 (0,5g) | graurotbraun | rotbraun | 8,87 |
| B13 : 1 g | ziegelrot | gelborange | 10,0 |
| B13 (0,5g) + A1 (0,5g) | braunorange | hennarot | 6,0 |
| B14 : 1 g | braunorange | gelb | 13,64 |
| B14 (0,5g) + A1 (0,5g) | orangebraun | orangebraun | 10,2 |
| B15 : 1 g | rotbraun | mattrotbraun | 8,9 |
| B15 (0,5g) + A1 (0,5g) | rotbraun | rotbraun | 8,4 |
| B16 : 1 g | graugrün | mattgrün | 9,15 |
| B16 (0,5g) + A1 (0,5g) | graurubin | mattrubin | 10,6 |
| B17 : 1 g | purpurgrau | mattviolett | 17,65 |
| B17 (0,5g) + A1 (0,5g) | graurot | graurot | 6,6 |
| B18 : 1 g | silbergrau | violettgrau | 11,2 |
| B18 (0,5g) + A1 (0,5g) | graurot | graurot | 2,30 |

Fortsetzung Tabelle I

| Direktzieher, Menge | Farbnuance Haarspitze | Haaransatz | Farb-Abstand DE |
|---|---|---|---|
| B19 : 1 g<br>B19 (0,5g) + A1 (0,5g) | graugrün<br>graurubin | grautürkis<br>graurubin | 19,9<br>7,74 |
| B20 : 1 g<br>B20 (0,5g) + A1 (0,5g) | graurubin<br>graurotbraun | graumagenta<br>rotbraun | 9,01<br>2,0 |
| B21 : 1 g<br>B21 (0,5g) + A1 (0,5g) | gelb<br>hellbraun | orangegelb<br>hellbraun | 10,0<br>7,3 |
| A1 (0,33 g)<br>B10 (0,33 g)<br>B17 (0,33 g) | braunviolett | braunviolett | 5,0 |
| A1 (0,33 g)<br>B1 (0,33 g)<br>B4 (0,33 g) | terracotta | rotbraun | 2,0 |
| A1 (0,33 g)<br>B1 (0,33 g)<br>B5 (0,33 g) | rotbraun | rotbraun | 3,9 |
| A1 (0,33 g)<br>B5 (0,33 g)<br>B8 (0,33 g) | violettbraun | violettbraun | 2,4 |
| A1 (0,25 g)<br>A2 (0,25 g)<br>B5 (0,25 g)<br>B9 (0,25 g) | graumagenta | graurubin | 3,8 |

**Patentansprüche**

1. Verwendung eines direktziehenden Farbstoffes (A) der Formel I

(I) 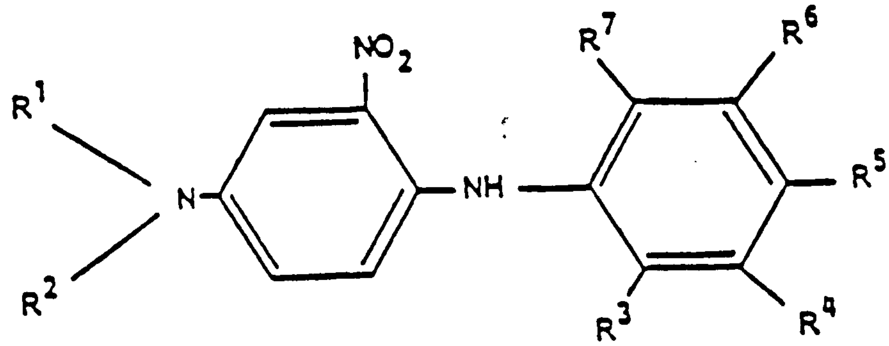

in der $R^1$ und $R^2$ Wasserstoff, Alkylgruppen mit 1 bis 4 C-Atomen oder Hydroxyalkylgruppen mit 2 bis 4 C-Atomen und eine der Gruppen $R^3$ bis $R^7$ eine $-SO_3H$ oder eine -COOH-Gruppe ist und die anderen Wasserstoff, Chlor, Alkylgruppen mit 1 bis 4 C-Atomen, Alkoxygruppen mit 1 bis 4 C-Atomen oder Gruppen der Formel $-NR^8R^9$ sind, worin $R^8$ und $R^9$ Wasserstoff, Alkylgruppen mit 1 bis 4 C-Atomen, Hydroxyalkylgruppen mit 2 bis 4 C-Atomen sind oder gemeinsam mit dem Stickstoffatom einen Piperidin-, Morpholin-, Piperazin- oder Pyrrolidinring bilden, ist oder eines seiner wasserlöslichen Salze, in Haarfärbemitteln mit einem Gehalt an wenigstens einem zweiten direktziehenden Farbstoff (B) einer anderen Struktur in einem kosmetischen Träger, zur Verbesserung der Gleichmäßigkeit der Haaranfärbung, insbesondere von durch reduktive oder oxidative Haarbehandlung geschädigtem Haar.

2. Verwendung nach Patentanspruch 1, dadurch gekennzeichnet, daß wenigstens ein zweiter direktziehender Farbstoff (B) aus der Gruppe der aromatischen Nitroverbindungen oder der Anthrachinonverbindungen enhalten ist.

3. Verwendung nach Patentanspruch 1 oder 2, dadurch gekennzeichnet, daß die direktziehenden Haarfarbstoffe insgesamt in einer Menge von 0,05 - 5 Gew.-% und die Farbstoffe (A) der Formel I zu den übrigen Farbstoffen (B) in einem Gewichtsverhältnis von A : B = 7 : 3 bis 3 : 1 enthalten sind.

4. Verwendung nach Patentanspruch 1 - 3, dadurch gekennzeichnet, daß der Träger eine Emulsion ist und 1 - 5 Gew.-% eines Fettalkohols mit 12 - 22 C-Atomen und 5 - 20 Gew.-% eines Anlagerungsproduktes von 5 - 20 Mol Ethylenoxid an ein Mol eines linearen, endständigen Alkylamins mit 12 - 22 C-Atomen, jeweils bezogen auf das Gewicht des gesamten Haarfärbemittels, enthält.

**Claims**

1. The use of a substantive dye (A) corresponding to formula I

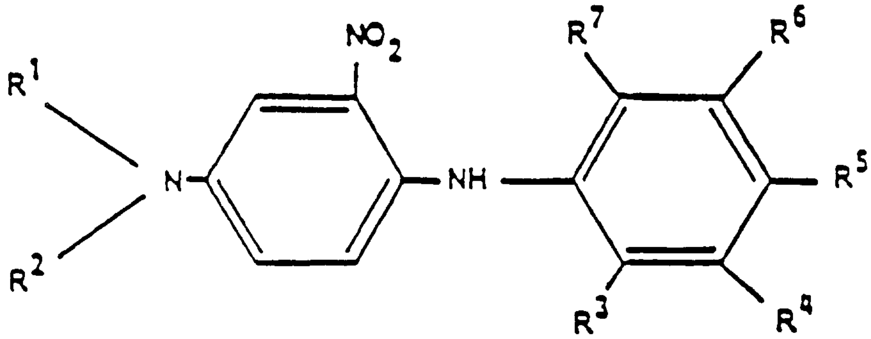 (I)

in which $R^1$ and $R^2$ are hydrogen, $C_{1-4}$ alkyl groups or $C_{2-4}$ hydroxyalkyl groups and one of the

substituents $R^3$ to $R^7$ is an $-SO_3H$ or a -COOH group and the other substituents are hydrogen, chlorine, $C_{1-4}$ alkyl groups, $C_{1-4}$ alkoxy groups or groups of the formula $-NR^8R^9$, where $R^8$ and $R^9$ are hydrogen, $C_{1-4}$ alkyl groups, $C_{2-4}$ hydroxyalkyl groups or, together with the nitrogen atom, form a piperidine, morpholine, piperazine or pyrrolidine ring,
or one of its water-soluble salts in hair-dyeing preparations containing at least asecond substantive dye (B) of a different structure in a cosmetic carrier for improving the uniformity of hair dyeing, particularly the dyeing of hair damaged by reductive or oxidative hair treatments.

**2.** The use claimed in claim 1, characterized in that at least a second substantive dye (B) from the group of aromatic nitro compounds or anthraquinone compounds is present.

**3.** The use claimed in claim 1 or 2, characterized in that the substantive hair dyes are present in a total quantity of 0.05 to 5% by weight, the ratio by weight of the dyes (A) corresponding to formula I to the other dyes (B) being 7:3 to 3:7.

**4.** The use claimed in claims 1 to 3, characterized in that the carrier is an emulsion and contains 1 to 5% by weight of a fatty alcohol containing 12 to 22 carbon atoms and 5 to 20% by weight of an adduct of 5 to 20 mol ethylene oxide with 1 mol of a linear terminal alkyl amine containing 12 to 22 carbon atoms, based on the weight of the hair-dyeing preparation as a whole.

## Revendications

**1.** Utilisation d'un colorant (A) montant directement sur la fibre répondant à la formule I

(I)

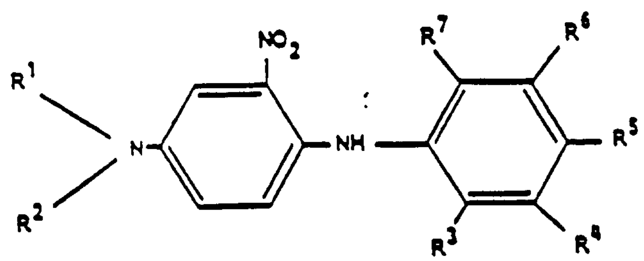

dans laquelle $R^1$ et $R^2$ représentent un atome d'hydrogène, des groupes alkyle contenant de 1 à 4 atomes de carbone ou des groupes hydroxyalkyle contenant de 2 à 4 atomes de carbone, et un des groupes $R^3$ à $R^7$ représente un groupe $-SO_3H$ ou un groupe -COOH et l'autre représente un atome d'hydrogène, un atome de chlore, des groupes alkyle contenant de 1 à 4 atomes de carbone, des groupes alcoxy contenant de 1 à 4 atomes de carbone ou encore des groupes de formule $-NR^8R^9$ où $R^8$ et $R^9$ rePrésentent un atome d'hydrogène, des groupes alkyle contenant de 1 à 4 atomes de carbone, des groupes hydroxyalkyle contenant de 2 à 4 atomes de carbone ou forment ensemble avec l'atome d'azote un noyau pipéridine, un noyau morpholine, un noyau pipérazine ou un noyau pyrrolidine, ou d'un de ses sels hydrosolubles, dans des teintures capillaires ayant une teneur en au moins un second colorant (B) montant directement sur la fibre d'une autre structure, dans un support cosmétique, pour améliorer l'uniformité de la coloration capillaire, en particulier de cheveux abîmés par un traitement capillaire par réduction ou par oxydation.

**2.** Utilisation selon la revendication 1; caractérisée en ce qu'est compris au moins un second colorant (B) montant directement sur la fibre choisi parmi le groupe des composés nitro aromatiques ou des composés d'anthraquinone.

**3.** Utilisation selon la revendication 1 ou 2, caractérisée en ce que les colorants capillaires montant directement sur la fibre sont compris au total en une quantité de 0,05-5% en poids, et le rapport pondéral A:B des colorants (A) de formule I aux colorants (B) restants est compris de 7:3 à 3:7.

**4.** Utilisation selon les revendications 1 à 3, caractérisée en ce que le support est une émulsion et contient de 1-5% en poids d'un alcool gras contenant de 12 à 22 atomes de carbone et de 5-20% en poids d'un produit d'addition de 5-20 modes d'oxyde d'éthylène à 1 mole d'une alkylamine linéaire

terminale contenant de 12 à 22 atomes de carbone, chaque fois rapportées au poids de la teinture capillaire totale.